# EUROPEAN PATENT APPLICATION

(11) **EP 2 450 042 A1**
(43) Date of publication of application: **09.05.2012**
(21) Application number: 10794201.3
(22) Date of filing: 30.06.2010
(51) Int. Cl.: A61K 31/58, A61P 3/08, A61P 3/10, C07J 17/00

(54) **GLUCOSE METABOLISM-IMPROVING AGENT AND GLUCOSE METABOLISM-IMPROVING COMPOSITION**

(30) Priority: 30.06.2009 JP 2009155914; 30.06.2009 JP 2009155957
(71) Applicant: Lion Corporation, Tokyo 130-8644 (JP)
(72) Inventor: IWASAKI, Hideaki, Tokyo 130-8644 (JP); KAMBAYASHI, Hiroaki, Tokyo 130-8644 (JP); NOMURA, Mitsuru, Tokyo 130-8644 (JP); SUZUKI, Naho, Tokyo 130-8644 (JP); KITAMURA, Kumiko, Tokyo 130-8644 (JP)
(74) Representative: Graf von Stosch, Andreas
(86) International application number: PCT/JP2010/061180
(87) International publication number: WO 2011/002033

(57) **Abstract**

A sugar metabolism-improving agent containing at least one of a compound having a structure expressed by the following Structural Formula (1) and a compound having a structure expressed by the following Structural Formula (2) and having an effect of suppressing an increase in the postprandial blood sugar level, an effect of decreasing the fasting blood sugar level, an effect of controlling sugar metabolism-related indices in serum, an effect of promoting intake of sugar into muscle cells, and an effect of promoting metabolism of sugar derived from a meal; a sugar metabolism-improving composition, an agent for suppressing an increase in the postprandial blood sugar level, an agent for decreasing the fasting blood sugar level, an agent for promoting intake of sugar, and food and drink each containing the sugar metabolism-improving agent.

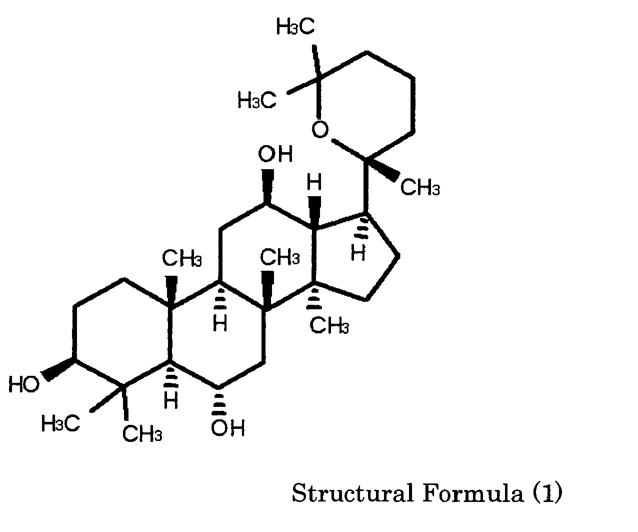

## Description

### Technical Field

The present invention relates to a sugar metabolism-improving agent containing at least one of panaxatriol (PT) and panaxadiol (PD) and to a sugar metabolism-improving composition containing the sugar metabolism-improving agent.

### Background Art

In Japan, the number of patients with diabetes is estimated to be about 8,200,000 while the number of those likely to develop diabetes is estimated to be about 10,500,000, and these numbers have been increasing year by year. Japanese have constitution easy to develop diabetes, in which foods are hardly consumed and easily stored in their bodies. The thrifty gene-carrying rate of Japanese is said to be 2 to 5 times that of western people. Thus, the prevention or treatment of diabetes is a critical issue.

The pathological condition of diabetes is classified into the three types: normal type, borderline type and diabetic type (according to the guideline of Japan Diabetes Society) and those likely to develop diabetes are patients of the borderline type.

The criteria for the diabetic type include: (1) the fasting blood sugar level is 126 mg/dL or higher; (2) the postprandial blood sugar level is 200 mg/dL or higher; (3) the blood sugar level is 200 mg/dL or higher measured two hours after the 75 g-glucose tolerance test; (4) typical symptoms of diabetes are observed (e.g., thirst, polyposia, polyuria and weight loss); (5) the level of HbAlc (glycohemoglobin) is 6.5% by mass or higher; and (6) diabetic retinopathy is surely observed.

The criteria for the borderline type include: (1) the fasting blood sugar level is 110 mg/dL or higher but lower than 126 mg/dL and (2) the blood sugar level is 140 mg/dL or higher but lower than 200 mg/dL measured two hours after the 75 g-glucose tolerance test.

Currently known therapeutic drugs for diabetes include: sulfonylurea agents (SU agents) and phenylalanine derivatives which promote secretion of insulin; α-glycosidase inhibitors which suppress absorption of sugar; biguanide (BG) drugs which suppress production of sugar in the liver; and thiazolidine derivatives which improve insulin resistance. There are a few drugs that have an effect of controlling both of the fasting blood sugar level and the postprandial blood sugar level. Also, these drugs are prescribed only when one has been diagnosed as diabetes. Thus, at present, there are only diet therapy and exercise therapy that can be performed to prevent those of the borderline type from suffering from diabetes.

Desirably, hyperglycemic condition caused by diabetes is drastically improved; i.e., not transiently but permanently in the constitution. Extra glucose in the body is taken in from blood to the liver and muscle where it is stored as glycogen. Thus, promotion of glycogen synthesis is thought to improve such hyperglycemic condition. Since 70% or higher of sugar metabolism is performed in the muscle, promoting intake of glucose into the muscle is expected to improve hyperglycemic condition.

In order to maintain constitution in which sugar metabolism is positively performed in the muscle, it is effective to improve diabetes not only by the aforementioned drugs but also through diet therapy. Besides the above drugs, there exist numerous drugs that are ingestible as foods and reduce the postprandial blood sugar level. Meanwhile, there are no compounds that are ingestible as foods and reduce the fasting blood sugar level; i.e., the compounds that reduce the fasting blood sugar level are all used only as drugs. Therefore, keen demand has arisen for provision of foods that reduce the fasting blood sugar level.

A glycoside (ginsenoside) contained in ginseng is known to have a blood sugar level-controlling effect (see PTL 1) and an anti-diabetic effect (see PTL 2). Also, protopanaxatriol (PPT) and protopanaxadiol (PPD), which are aglycons remaining after removal of the sugar moiety from the above glycoside, are known to have various effects such as an anti-cancer effect (see PTLs 3 and 4), an anti-inflammatory effect to skin diseases (see PTL 5), an effect of activating PPARγ which regulates the expression of genes playing an important role in fat metabolism and sugar metabolism (see PTL 6) and an effect of suppressing the synthesis of a heat shock protein (HSP) involved in autoimmune diseases (see PTL 7).

Although protopanaxatriol (PPT) and protopanaxadiol (PPD) are white powder and insoluble to water, their solubility can be improved by the addition of an organic solvent. However, protopanaxatriol (PPT) and protopanaxadiol (PPD) each have an unstable structure and disadvantageously, they are rapidly decomposed in a liquid system, especially in a low-pH system. Even in the powder form, they are progressively decomposed day by day at a temperature equal to or higher than ambient temperature, which is problematic.

Therefore, at present, keen demand has arisen for provision of a compound which has a blood sugar level-controlling effect, a sugar metabolism-improving effect, high safety and high stability as well as which can be given as food and drink.

### Citation List

### Patent Literature

PTL 1: Japanese Patent Application Laid-Open (JP-A) No. 2008-533132
PTL 2: JP-A No. 61-24597
PTL 3: JP-A No. 2005-504799
PTL 4: JP-A No. 58-57399
PTL 5: JP-A No. 2007-008896
PTL 6: Korean Patent Application Laid-Open No. 10-2006-0131012
PTL 7: JP-A No. 09-241166

### Summary of Invention

### Technical Problem

The present invention aims to solve the existing problems pertinent in the art and achieve the following objects. Specifically, an object of the present invention is to provide a sugar metabolism-improving agent which has an excellent blood sugar level-controlling effect, and an excellent sugar metabolism-improving effect, high safety and high stability as well as which can be given as food and drink; and a sugar metabolism-improving composition containing the sugar metabolism-improving agent.

### Solution to Problem

The present inventors conducted extensive studies to solve the above existing problems and have obtained the following finding. That is, they have found that a sugar metabolism-improving agent containing at least one of panaxatriol (PT) and panaxadiol (PD) promotes intake of sugar into muscle cells has an effect of suppressing an increase in the postprandial blood sugar level, an effect of decreasing the fasting blood sugar level, an effect of controlling sugar metabolism-related indices, and an effect of promoting metabolism of sugar derived from a meal. The present invention has been accomplished on the basis of this finding.

The present invention is based on the finding obtained by the present inventors. Means for solving the existing problems are as follows.
<1> A sugar metabolism-improving agent including:
   at least one of a compound having a structure expressed by the following Structural Formula (1) and a compound having a structure expressed by the following Structural Formula (2). 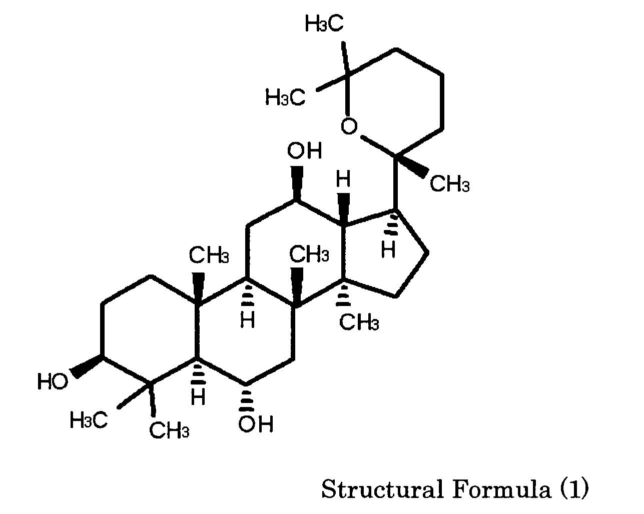 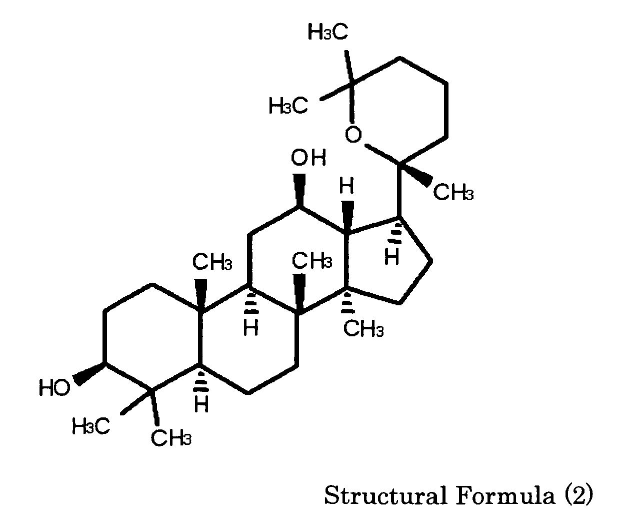
<2> The sugar metabolism-improving agent according to <1>, wherein the sugar metabolism-improving agent has an effect of suppressing an increase in a postprandial blood sugar level.
<3> The sugar metabolism-improving agent according to <1> or <2>, wherein the sugar metabolism-improving agent has at least one of an effect of decreasing a fasting blood sugar level and an effect of controlling a sugar metabolism-related index in serum.
<4> The sugar metabolism-improving agent according to any one of <1> to <3>, wherein the sugar metabolism-improving agent has an effect of promoting metabolism of sugar derived from a meal.
<5> The sugar metabolism-improving agent according to any one of <1> to <4>, wherein the sugar metabolism-improving agent has an effect of promoting intake of sugar into muscle cells.
<6> The sugar metabolism-improving agent according to any one of <1> to <5>, wherein the sugar metabolism-improving agent exhibits a sugar metabolism-improving effect regardless of whether or not the sugar metabolism-improving agent is given at the same time as a meal.
<7> The sugar metabolism-improving agent according to any one of <1> to <6>, wherein an amount of the sugar metabolism-improving agent given per day is at least 1 mg.
<8> A sugar metabolism-improving composition including:
   the sugar metabolism-improving agent according to any one of <1> to <7>.
<9> An agent for suppressing an increase in a postprandial blood sugar level including:
   the sugar metabolism-improving agent according to any one of <1> to <7>.
<10> An agent for decreasing a fasting blood sugar level including:
   the sugar metabolism-improving agent according to any one of <1> to <7>.
<11> An agent for promoting intake of sugar into muscle cells including:
   the sugar metabolism-improving agent according to any one of <1> to <7>.
<12> Food and drink including:
   the sugar metabolism-improving agent according to any one of <1> to <7>.

### Advantageous Effects of Invention

The present invention can provide: a sugar metabolism-improving agent which has an excellent blood sugar level-controlling effect, an excellent sugar metabolism-improving effect, high safety and high stability as well as which can be given as food and drink; and a sugar metabolism-improving composition containing the sugar metabolism-improving agent. These can solve the above existing problems and achieve the above objects.

### Brief Description of Drawings

Fig. 1 is a graph of changes in fasting blood sugar level in the test of PT intake for human in Example 5, where the vertical axis indicates blood sugar levels (mg/dL) and the horizontal axis indicates the number of days which have passed since the initiation of PT intake.
Fig. 2 is a graph of changes in postprandial blood sugar level in the placebo-given group of the test of PT intake for human in Example 5, where the vertical axis indicates blood sugar levels (mg/dL) and the horizontal axis indicates the time (min) which has passed since the meal.
Fig. 3 is a graph of changes in postprandial blood sugar level in the panaxatriol (PT)-given group of the test of PT intake for human in Example 5, where the vertical axis indicates blood sugar levels (mg/dL) and the horizontal axis indicates the time (min) which has passed since the meal.
Fig. 4 is a graph of changes in fasting blood sugar level in the test of PD intake for human in Example 6, where the vertical axis indicates blood sugar levels (mg/dL) and the horizontal axis indicates the number of days which have passed since the initiation of PD intake.
Fig. 5 is a graph of changes in postprandial blood sugar level in the placebo-given group of the test of PD intake for human in Example 6, where the vertical axis indicates blood sugar levels (mg/dL) and the horizontal axis indicates the time (min) which has passed since the meal.
Fig. 6 is a graph of changes in postprandial blood sugar level in the panaxadiol (PD)-given group of the test of PD intake for human in Example 6, where the vertical axis indicates blood sugar levels (mg/dL) and the horizontal axis indicates the time (min) which has passed since the meal.
Fig. 7A is a graph of changes over time in the amount of ¹³CO₂ excreted in the groups of Example 7, where the vertical axis indicates the amount of excreted ¹³CO₂ contained in exhaled gas (amount by volume %) and the horizontal axis indicates the time (min) which has passed since the administration of sugar-U-¹³C₆.
Fig. 7B is a graph of cumulative amounts of excreted ¹³CO₂ in the groups of Example 7, where the vertical axis indicates AUC (the increment in the amount of ¹³CO₂ excreted).

### Description of Embodiments

### (Sugar metabolism-improving agent)

A sugar metabolism-improving agent of the present invention contains at least one of a compound having a structure expressed by the following Structural Formula (1) and a compound having a structure expressed by the following Structural Formula (2).

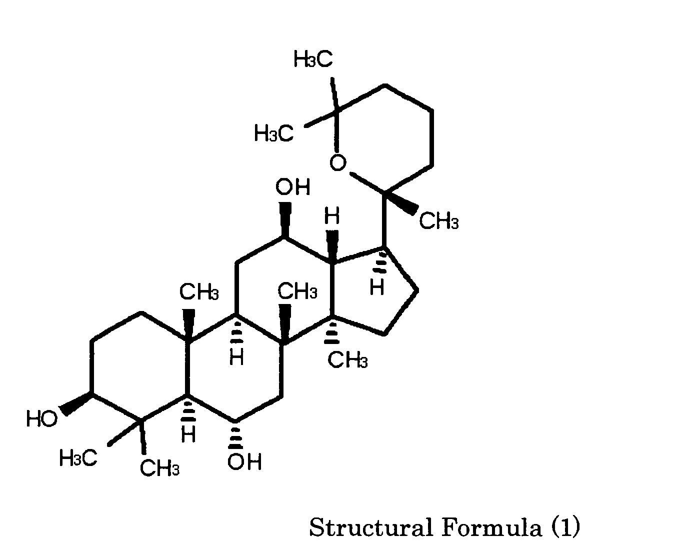

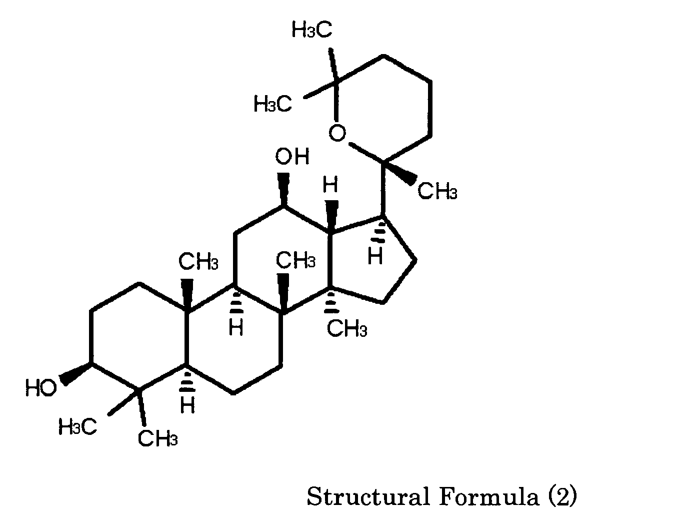

### <Compound having Structural Formula (1) and compound having Structural Formula (2)>

The compound having the above Structural Formula (1) is a compound belonging to dammarane-type triterpenes. Hereinafter, this compound may be referred to as "panaxatriol (PT)." The compound having the above Structural Formula (2) is also a compound belonging to dammarane-type triterpenes. Hereinafter, this compound may be referred to as "panaxadiol (PD)."

Panaxatriol (PT) and panaxadiol (PD) are each an aglycon formed as follows: the sugar moiety is removed from a plant-origin saponin (glycoside) and the side chains are ring-closed.

### -Method for obtaining panaxatriol (PT) and panaxadiol (PD)-

The method for obtaining panaxatriol (PT) and panaxadiol (PD) is not particularly limited and may be appropriately selected depending on the intended purpose. Examples thereof include a method in which commercially available products of panaxatriol (PT) and panaxadiol (PD) are used, a method in which panaxatriol (PT) and panaxadiol (PD) are obtained through synthesis, and a method in which panaxatriol (PT) and panaxadiol (PD) are obtained from plants.

The plants are not particularly limited and may be appropriately selected depending on the intended purpose. Ginseng belonging to the family Araliaceae is preferred, with *Panaxn otoginseng* being more preferred.

Saponin derived from the *Panax notoginseng* is not particularly limited and may be appropriately selected depending on the intended purpose. Examples thereof include ginsenoside-Rg₁, notoginsenoside-R₁, ginsenoside-Re, ginsenoside-Rb₁, ginsenoside-Rd and ginsenoside-Rc.

Next will be described one exemplary method for obtaining panaxatriol (PT) and panaxadiol (PD) derived from the ginseng belonging to the family Araliaceae. The method for obtaining panaxatriol (PT) and panaxadiol (PD) derived from the ginseng belonging to the family Araliaceae is not particularly limited and may be appropriately selected depending on the intended purpose. Examples thereof include a method in which panaxatriol (PT) and panaxadiol (PD) are extracted and/or purified from the ginseng belonging to the family Araliaceae.

The method for obtaining panaxatriol (PT) and panaxadiol (PD) through extraction is not particularly limited and may be appropriately selected depending on the intended purpose. Examples thereof include a method in which powder of *Panax notoginseng* is extracted with a water-ethanol solution.

The mixing ratio between water and ethanol in the water-ethanol solution is not particularly limited and may be appropriately selected depending on the intended purpose. The mixing ratio of water:ethanol (V/V) is preferably 9:1 to 2:1, more preferably 3:1.

In the above extraction, preferably, hydrochloric acid is added the water-ethanol solution for performing acid hydrolysis. This is because an extract containing at least one of panaxatriol (PT) and panaxadiol (PD) at a high concentration can be obtained.

The concentration of the hydrochloric acid in the water-ethanol solution is not particularly limited and may be appropriately selected depending on the intended purpose. It is preferably 0.04% by mass to 16% by mass, more preferably 2% by mass to 12% by mass.

The temperature at which the acid hydrolysis is performed is not particularly limited and may be appropriately selected depending on the intended purpose. It is preferably 60°C to 100°C, more preferably 70°C to 90°C.

The time for which the acid hydrolysis is performed is not particularly limited and may be appropriately selected depending on the intended purpose. It is preferably 0.5 hours to 24 hours, more preferably 2 hours to 8 hours.

If necessary, the *Panax notoginseng* hydrolysis liquid obtained through the acid hydrolysis is neutralized with sodium hydroxide and is treated so that the ethanol concentration is decreased. Then, the resultant liquid is filtrated through aspiration or other methods, and the residue is subjected to a drying treatment such as freeze drying, reduced-pressure drying or spray drying, whereby at least one of panaxatriol (PT) and panaxadiol (PD) derived from *Panax notoginseng* can be obtained.

The method for obtaining panaxatriol (PT) and panaxadiol (PD) through purification is not particularly limited and may be appropriately selected depending on the intended purpose. Examples thereof include a method in which panaxatriol (PT) and panaxadiol (PD) are purified with a silica gel column.

The method in which panaxatriol (PT) and panaxadiol (PD) are purified with a silica gel column is not particularly limited and may be appropriately selected depending on the intended purpose. In one employable method, the product obtained through the acid hydrolysis is dissolved in ethanol to prepare an ethanol solution containing it at a concentration of 1% by mass to 5% by mass; the ethanol solution is treated with filter paper or a centrifuge to remove insoluble matter; the thus-treated solution is 5- to 10-fold concentrated with a rotary evaporator; and the concentrated liquid is applied to a glass column packed with silica gel (e.g., SILICA GEL 60N, product of KANTO CHEMICAL CO., LTD.) using as an eluent chloroform:ethanol = 10:1 (V/V) (column separation/collection).

Through normal-phase TLC using a developing solvent of chloroform:ethanol = 10:1 (V/V), a fraction corresponding to a Rf value of 0.4 is concentrated whereby panaxatriol (PT) of high purity can be obtained. Similarly, through normal-phase TLC using a developing solvent of chloroform:ethanol = 10:1 (V/V), a fraction corresponding to a Rf value of 0.6 is concentrated whereby panaxadiol (PD) of high purity can be obtained.

### <Intake>

The method, amount, frequency, time, and target of intake of the sugar metabolism-improving agent are not particularly limited and may be appropriately selected depending on the intended purpose.

The method of intake thereof is not particularly limited and may be appropriately selected depending on the intended purpose. A method of orally giving the sugar metabolism-improving agent is preferred, since users can easily continue to take in it.

The amount of intake thereof is not particularly limited and may be appropriately determined considering various factors of target individuals such as their age, body weight, constitution, symptoms and concomitant use of a drug containing other active ingredients. The daily intake (amount of the sugar metabolism-improving agent given per day) is preferably at least 1 mg, more preferably 2 mg to 20 mg. The daily intake within this preferred range is advantageous in that it is possible to control both the postprandial blood sugar level and the fasting blood sugar level as well as to improve intake suitability.

The frequency of intake thereof is not particularly limited and may be appropriately selected depending on the intended purpose. The sugar metabolism-improving agent is preferably given once a day, since such a frequency is convenient for users.

The time of intake thereof is not particularly limited and may be appropriately selected depending on the intended purpose. In order to reduce intake-related bothers of users, the time of intake thereof should not be limited to a certain time such as the same time as a meal or after a meal. It is preferred that the sugar metabolism-improving agent exhibit its sugar metabolism-improving effects even when it is not given at the same time as a meal. However, so long as the form of the sugar metabolism-improving agent is a dosage form which can be given as an ordinary food together with a meal without involving bothers, the sugar metabolism-improving effects can be obtained without depending on the time when the sugar metabolism-improving agent is given. In other words, the time when the sugar metabolism-improving agent is given is not limited to the same time as a meal.

Among the animal species that can be targets of intake thereof, the sugar metabolism-improving agent is applied suitably to humans. However, so long as the effects of the sugar metabolism-improving agent can be obtained, the sugar metabolism-improving agent may also be applied to non-human animals such as mice, rats, hamsters, birds, dogs, cats, sheep, goats, bovine, pigs and monkeys.

### <Sugar metabolism-improving effects>

The sugar metabolism-improving effects of the sugar metabolism-improving agent are not particularly limited and may be appropriately selected depending on the intended purpose. The sugar metabolism-improving agent preferably has at least one of an effect of suppressing an increase in the postprandial blood sugar level by promoting intake of sugar into cells, an effect of decreasing the fasting blood sugar level, and an effect of promoting metabolism of sugar derived from a meal.

### -Intake of sugar into cells-

The cells are not particularly limited and may be appropriately selected depending on the intended purpose. Examples thereof include muscle cells, liver cells and fat cells, with muscle cells being preferred. The muscle cells are responsible for 70% or higher of sugar metabolism and thus are expected to effectively function as an organ responsible for intake of sugar. In contrast, in the case of the liver cells and the fat cells, there is a risk of developing symptoms such as fatty liver and obesity. Although hyperglycemic condition is expected to be improved when sugar is taken into the liver cells and the fat cells, it is desirable that sugar be taken into the muscle cells mainly, if possible.

The mechanism by which the intake of sugar is promoted is not clear in detail. Presumably, it is due to the fact that the sugar metabolism-improving agent promotes translocation, onto cell membrane, of GLUT4 which is localized in cells.

### --Method for evaluating promotion of sugar intake--

The method for evaluating promotion of sugar intake is not particularly limited and may be appropriately selected depending on the intended purpose. In one employable method, the sugar metabolism-improving agent is added to a medium containing culture cells so that the culture cells are sensitized with the sugar metabolism-improving agent for a certain period; the culture cells are allowed to take in labeled sugar and then are lysed; and the sugar intake is measured based on the label in the culture cells.

The culture cells are not particularly limited and may be appropriately selected depending on the intended purpose. Examples thereof include L6 cells derived from rat skeletal muscle and C2C12 cells derived from mouse skeletal muscle.

The medium used for culturing the L6 cells is not particularly limited and may be appropriately selected depending on the intended purpose. Examples thereof include DMEM (Dulbecco's modified Eagle medium) containing 10% by mass FBS (fetal bovine serum) and 1% by mass AB (anti-biotic solution). The method for inducing differentiation of the L6 cells is not particularly limited and may be appropriately selected depending on the intended purpose. Examples thereof include a method in which the L6 cells are cultured in MEM containing 2% by mass FBS and 1% by mass AB.

The label of sugar is not particularly limited and may be appropriately selected depending on the intended purpose. Examples thereof include a RI label. The method for making the RI-labeled sugar taken is not particularly limited and may be appropriately selected depending on the intended purpose. Examples thereof include a method in which the RI-labeled sugar is added to the medium. The sugar is not particularly limited and may be appropriately selected depending on the intended purpose. Examples thereof include glucose.

The method for lysing the cells is not particularly limited and may be appropriately selected depending on the intended purpose. Examples thereof include a method using 0.05N sodium hydroxide.

When the label of the sugar is a RI label, the method for measuring its radioactivity is not particularly limited and may be appropriately selected depending on the intended purpose. In one employable method, scintillation cocktail PICOFLOUR (product of PerkinElmer Co., Ltd.) is added to a vial containing lysed cells recovered, followed by measurement with a scintillation counter.

### -Postprandial blood sugar level-

The postprandial blood sugar level refers to a blood sugar level that is measured 30 min to 120 min after a meal.

The postprandial blood sugar level may be appropriately selected depending on the age and the period of time having passed after a meal. In human, the postprandial blood sugar level that is measured 30 min after a meal is preferably lower than 180 mg/dL, and the postprandial blood sugar level that is measured 120 min after a meal is preferably lower than 140 mg/dL.

### --Method for evaluating postprandial blood sugar level--

The method for evaluating the postprandial blood sugar level is not particularly limited and may be appropriately selected depending on the intended purpose. Examples thereof include an evaluation method using a model mouse of hyperglycemia and an evaluation method by loading starch foods to human.

The method for measuring the blood sugar level is not particularly limited and may be appropriately selected depending on the intended purpose. Examples thereof include a method in which the blood sugar level is measured with a simplified blood sugar meter such as FREESTYLE (product of NIPRO Co.) and a method in which the blood sugar level is measured with CYCLIC GB SENSOR (product of Sanko Junyaku Co., Ltd.).

### ---Evaluation method using a model mouse of hyperglycemia---

The evaluation method using a model mouse of hyperglycemia is not particularly limited and may be appropriately selected depending on the intended purpose. In one exemplary method, the mouse is bred for a certain period with feed containing the sugar metabolism-improving agent and high fat diet, and then measured for the blood sugar level after the breeding.

The model mouse of hyperglycemia is not particularly limited and may be appropriately selected depending on the intended purpose. Examples thereof include KKAy mouse (product of CLEA Japan, Inc.) and ZDF rat (product of Charles River Laboratories Japan, Inc.).

The high fat diet is not particularly limited and may be appropriately selected depending on the intended purpose. Examples thereof include commercially available products such as Quick Fat (product of CLEA Japan, Inc.).

The period for breeding is not particularly limited and may be appropriately selected depending on the intended purpose. It is, for example, 4 days to 35 days.

### ---Evaluation method by loading starch foods to human---

The evaluation method by loading starch foods to human is not particularly limited and may be appropriately selected depending on the intended purpose. In one exemplary method, predetermined meals are given for a certain test period to human subjects with a high fasting blood sugar level (120 mg/dL to 140 mg/dL); then the sugar metabolism-improving agent is given to them once a day at a time different from the time of a meal; and then a change in the blood sugar level is measured every 30 min for 120 min after a meal.

The test period is not particularly limited and may be appropriately selected depending on the intended purpose. It is, for example, 4 weeks to 16 weeks.

The predetermined meal is not particularly limited and may be appropriately selected depending on the intended purpose. Preferred examples thereof include meals containing a large amount of starch, such as rice and pasta.

### -Fasting blood sugar level-

The fasting blood sugar level refers to a blood sugar level of blood sampled from a subject receiving neither foods nor drinks after arising in the morning. Strictly, it is desirably a blood sugar level of blood sampled after 10 hours or longer has passed after a meal. The fasting blood sugar level may be appropriately selected depending on, for example, the age of the subject. In human, it is preferably lower than 110 mg/dL.

### --Method for evaluating fasting blood sugar level--

The method for evaluating the fasting blood sugar level is not particularly limited and may be appropriately selected depending on the intended purpose. For example, the fasting blood sugar level can be evaluated by the same method as the evaluation method by loading starch foods to human except that the blood sugar level is measured during fasting.

### -Sugar metabolism-related index-

The sugar metabolism-related index is not particularly limited and may be appropriately selected depending on the intended purpose. Examples thereof include hematological indices.

The hematological indices are not particularly limited and may be appropriately selected depending on the intended purpose. Examples thereof include HbAlc (glycohemoglobin), glycoalbumin, 1.5AG (1,5-anhydroglucitol) and insulin.

The method for measuring the hematological indices is not particularly limited and may be appropriately selected depending on the intended purpose. In one exemplary method, the hematological indices can be measured by a routine method of a medical facility using the blood sampled in the evaluation method by loading starch foods to human.

### --HbAlc--

The HbAlc (glycohemoglobin) is a substance in which hemoglobin (Hb) in erythrocyte is bound to glucose. That is, the HbAlc level increases with increasing of the blood sugar level. Since the reaction rate of binding between hemoglobin (Hb) and glucose is low, the HbAlc level reflects an average blood sugar level for the past one to two months without depending on temporary physiological conditions.

The normal level of HbAlc is 4.3% by mass to 5.8% by mass. When the HbAlc level is 6.5% by mass or higher, there is a quite high possibility that the subject suffers from diabetes.

### --Glycoalbumin--

The glycoalbumin is a substance in which albumin in blood is bound to glucose. That is, the glycoalbumin level increases with increasing of the blood sugar level.

Since the albumin has a shorter half life than the HbAlc, the glycoalbumin level reflects an average blood sugar level at a past time closer to the present than in the HbAlc; i.e., one week to two weeks ago.

The normal level of glycoalbumin is 11.6% by mass to 16.4% by mass.

### --1-5AG--

The 1-5AG is a polyol having a similar structure to glucose and is abundant in the body. The 1.5AG is supplied from foods and extra 1.5AG is excreted in urine. In normal conditions, the 1.5AG is reabsorbed in the renal tubule. When glucose is excreted (urinary sugar) with increasing of the blood sugar level, the reabsorption of the 1.5AG is competitively inhibited. As a result, the 1.5AG is lost in urine whereby the blood 1.5AG level decreases. In this way, since the 1.5AG increases or decreases due to the occurrence of urinary sugar, it can be an index of the blood sugar level at the present or immediate past time.

Here, when the HbAlc level is 10% by mass or higher, the 1.5AG is excreted outside of the body. Thus, in this case, it is suitable that the HbAlc is used as an index.

The normal level of the 1.5AG is 14 µg/mL to 46 µg/mL.

### -Effect of promoting metabolism of sugar derived from a meal-

The sugar derived from a meal is not particularly limited and may be appropriately selected depending on the intended purpose. Examples thereof include sucrose, maltose, lactose, glucose, fructose and galactose.

The method for evaluating the effect of promoting metabolism of sugar is not particularly limited and may be appropriately selected depending on the intended purpose. Examples thereof include a method by measuring the amount of carbon dioxide excreted.

### <Application>

The sugar metabolism-improving agent has an excellent effect of suppressing an increase in the postprandial blood sugar level, an excellent effect of decreasing the fasting blood sugar level, an excellent effect of controlling the sugar metabolism-related indices, an excellent effect of promoting intake of sugar into muscle cells, and an excellent effect of promoting metabolism of sugar derived from a meal. Thus, the sugar metabolism-improving agent can suitably be used as the below-described sugar metabolism-improving composition, an agent for suppressing an increase in the postprandial blood sugar level, an agent for decreasing the fasting blood sugar level, and an agent for promoting sugar intake.

### (Sugar metabolism-improving composition)

A sugar metabolism-improving composition of the present invention contains the above-described sugar metabolism-improving agent; and, if necessary, further contains other ingredients.

The amount of the sugar metabolism-improving agent contained in the sugar metabolism-improving composition is not particularly limited and may be appropriately selected depending on the intended purpose. Also, the sugar metabolism-improving composition may be the sugar metabolism-improving agent itself.

The other ingredients contained in the sugar metabolism-improving composition are not particularly limited, so long as the effects of the present invention are not impeded, and may be appropriately selected depending on the intended purpose. Examples thereof include pharmacologically acceptable carriers such as ethanol, water and starch, and supplemental materials or additives used in the below-described food and drink.

The amount of the other ingredients contained in the sugar metabolism-improving composition is not particularly limited and may be appropriately selected depending on the intended purpose.

### <Use>

The sugar metabolism-improving composition may be used alone or in combination of two or more thereof. Alternatively, the sugar metabolism-improving composition may be used in combination with a drug containing other active ingredients. Furthermore, the sugar metabolism-improving composition may be incorporated before use into a drug containing other active ingredients.

### <Application>

Regarding applications of the sugar metabolism-improving composition, the sugar metabolism-improving composition can suitably be used for the prevention or treatment of diabetes, for example. Also, the sugar metabolism-improving composition can suitably be used in the below-described food and drink.
(Agent for suppressing an increase in the postprandial blood sugar level, agent for decreasing the fasting blood sugar level, and agent for promoting sugar intake)

### <Agent for suppressing an increase in the postprandial blood sugar level>

An agent of the present invention for suppressing an increase in the postprandial blood sugar level contains the above-described sugar metabolism-improving agent; and, if necessary, further contains other ingredients.

The amount of the sugar metabolism-improving agent contained in the agent for suppressing an increase in the postprandial blood sugar level is not particularly limited and may be appropriately selected depending on the intended purpose. Also, the agent for suppressing an increase in the postprandial blood sugar level may be the sugar metabolism-improving agent itself.

The other ingredients contained in the agent for suppressing an increase in the postprandial blood sugar level are not particularly limited and may be appropriately selected depending on the intended purpose. For example, depending on the dosage form of the agent for suppressing an increase in the postprandial blood sugar level, the other ingredients may be appropriately selected from pharmacologically acceptable carriers such as ethanol, water and starch.

The amount of the other ingredients contained in the agent for suppressing an increase in the postprandial blood sugar level is not particularly limited and may be appropriately selected depending on the intended purpose.

### <Agent for decreasing the fasting blood sugar level>

An agent of the present invention for decreasing the fasting blood sugar level contains the above-described sugar metabolism-improving agent; and, if necessary, further contains other ingredients.

The amount of the sugar metabolism-improving agent contained in the agent for decreasing the fasting blood sugar level is not particularly limited and may be appropriately selected depending on the intended purpose. Also, the agent for decreasing the fasting blood sugar level may be the sugar metabolism-improving agent itself.

The other ingredients contained in the agent for decreasing the fasting blood sugar level are not particularly limited and may be appropriately selected depending on the intended purpose. For example, depending on the dosage form of the agent for decreasing the fasting blood sugar level, the other ingredients may be appropriately selected from pharmacologically acceptable carriers such as ethanol, water and starch.

The amount of the other ingredients contained in the agent for decreasing the fasting blood sugar level is not particularly limited and may be appropriately selected depending on the intended purpose.

### <Agent for promoting sugar intake>

An agent of the present invention for promoting sugar intake contains the above-described sugar metabolism-improving agent; and, if necessary, further contains other ingredients.

The amount of the sugar metabolism-improving agent contained in the agent for promoting sugar intake is not particularly limited and may be appropriately selected depending on the intended purpose. Also, the agent for promoting sugar intake may be the sugar metabolism-improving agent itself.

The other ingredients contained in the agent for promoting sugar intake are not particularly limited and may be appropriately selected depending on the intended purpose. For example, depending on the dosage form of the agent for promoting sugar intake, the other ingredients may be appropriately selected from pharmacologically acceptable carriers such as ethanol, water and starch.

The amount of the other ingredients contained in the agent for promoting sugar intake is not particularly limited and may be appropriately selected depending on the intended purpose.

### <Use>

The agent for suppressing an increase in the postprandial blood sugar level, the agent for decreasing the fasting blood sugar level, or the agent for promoting sugar intake may be used alone or in combination of two or more thereof. Alternatively, the agent for suppressing an increase in the postprandial blood sugar level, the agent for decreasing the fasting blood sugar level, or the agent for promoting sugar intake may be used in combination with a drug containing other active ingredients. Furthermore, the agent for suppressing an increase in the postprandial blood sugar level, the agent for decreasing the fasting blood sugar level, or the agent for promoting sugar intake may be incorporated before use into a drug containing other active ingredients.

### <Application>

Regarding applications of the agent for suppressing an increase in the postprandial blood sugar level, the agent for decreasing the fasting blood sugar level, or the agent for promoting sugar intake, the agent for suppressing an increase in the postprandial blood sugar level, the agent for decreasing the fasting blood sugar level, or the agent for promoting sugar intake can suitably be used for the prevention or treatment of diabetes, for example. Also, the agent for suppressing an increase in the postprandial blood sugar level, the agent for decreasing the fasting blood sugar level, or the agent for promoting sugar intake can suitably be used in the below-described food and drink.

### (Food and drink)

Food and drink of the present invention contain the above-described sugar metabolism-improving agent; and, if necessary, further contain other ingredients.

Here, the "food and drink" refers to those which are less harmful to human health and which are given orally or through the gastrointestinal tract in the ordinary social life. They are not limited to drugs, quasi drugs and foods within the administrative boundaries, but include a wide variety of orally-given common foods, healthy foods, health-promoting foods, quasi drugs and drugs.

The amount of the sugar metabolism-improving agent contained in the food and drink is not particularly limited. Depending on the types of the food and drink, the sugar metabolism-improving agent can appropriately be incorporated in such an amount that the effects of the present invention are not impeded.

The food and drink may contain only the sugar metabolism-improving agent or may be the sugar metabolism-improving agent itself.

### <Types of food and drink>

The types of the food and drink are not particularly limited and may be appropriately selected depending on the intended purpose. Examples thereof include drinks such as refreshing drinks, carbonated drinks, energy drinks, fruit drinks and lactic drinks; frozen desserts such as ice cream, ice sherbet and ice shavings; noodles such as buckwheat noodles, wheat noodles, vermicelli, coats of Chinese dumplings, coats of pork dumplings, Chinese noodles and instant noodles; snacks such as candies, gum, chocolate, tabletted snacks, munches, biscuits, jelly, jam, cream, baked confectionery and bread; marine products such as crab, salmon, Japanese littleneck, tuna, sardine, shrimps, prawns, bonito, mackerel, whale, oyster, saury, squid, bloody clam, scallop, abalone, sea chestnut, salmon caviar and *Sulculus diversicolor supertexta*; marine/livestock processed foods such as fish minced and steamed, ham and sausage; dairy products such as processed milk and fermented milk; fats and oils or processed foods thereof such as salad oil, *Tempura* oil, margarine, mayonnaise, shortening, whip cream and dressing; seasonings such as sauce and basting; retort pouch foods such as curry, stew, *Oyako-don* (a bowl of rice topped with boiled chicken and eggs), rice porridge, *Zosui* (rice soup), *Chuka-don* (a bowl of rice with a chop-suey-like mixture on it), *Katsu-don* (a rice bowl with pork cutlets), *Ten-don* (a tempura rice bowl), *Una-don* (an eel rice bowl), *hayashi rice* (hashed beef with rice), *Oden* (a dish containing several ingredients such as boiled eggs and radish), mapo doufu, *Gyu-don* (a beef rice bowl), meat sauce, egg soup, rice omelet, Chinese dumplings, pork dumplings, hamburger steak and meat balls; and healthy foods in various forms, dietary supplements, pharmaceutical drugs and quasi drugs.

### <Other ingredients>

The other ingredients contained in the food and drink are not particularly limited and may be appropriately selected depending on the intended purpose. Examples thereof include supplemental materials or additives commonly used for producing food and drink.

The supplemental materials or additives are not particularly limited and may be appropriately selected depending on the intended purpose. Examples thereof include glucose, fructose, sucrose, maltose, sorbitol, stevioside, rubusoside, corn syrup, lactose, citric acid, tartaric acid, malic acid, succinic acid, lactic acid, L-ascorbic acid, dl-α-tocopherol, sodium erythorbate, glycerin, propylene glycol, glycerin fatty acid esters, polyglycerin fatty acid esters, sucrose fatty acid esters, sorbitan fatty acid esters, gum arabic, carrageenan, casein, gelatin, pectin, agar, B vitamins, nicotinic-acid amide, calcium pantothenate, amino acids, calcium salts, dyes, perfumes and preservatives.

The amount of the other ingredients contained in the food and drink is not particularly limited and may be appropriately selected depending on the intended purpose.

### Examples

The present invention will next be described in more detail by way of Examples, which should not be construed as limiting the present invention thereto.

### (Example 1: Effect of decreasing the blood sugar level of a model mouse of hyperglycemia using PT-containing feed)

### <Method>

Panaxatriol (PT) (product of LKT Laboratories, Inc.) was mixed with a commercially available high fat diet (trade name: Quick Fat, product of CLEA Japan, Inc.) in amounts shown in the following Table 1. Each of the thus-prepared feeds was given ad libitum to model mice of hyperglycemia (KKAy mice (product of CLEA Japan, Inc.), eight weeks old, 5 mice/group) to breed them for five days (hereinafter these groups may be referred to as "PT-containing group"). Mice of a control group were given a panaxatriol (PT)-free feed (hereinafter this group may be referred to as "PT-free group").

The blood sugar level was measured at 10 a.m. before breeding of the panaxatriol (PT)-containing high fat diet (initial value) and after breeding thereof (post-breeding). CYCLIC GB SENSOR (product of Sanko Junyaku Co., Ltd.) was used for the measurement of the blood sugar level. The values shown in the following Table 1 are calculated on the basis of the average value of two values obtained by measuring each individual twice for the postprandial blood sugar level. Notably, statistical analysis was performed using the Dunnett's multiple test.

**Table 1**

| | PT-free group (control) | PT-containing groups | | |
|---|---|---|---|---|
| Amount of PT added | - | 0.0001% by mass | 0.001% by mass | 0.01% by mass |
| Blood sugar level (initial value) | 448±31 mg/dl | 455±39 mg/dl | 451±34 mg/dl | 451±38 mg/dl |
| Blood sugar level (post-breeding) | 531±44 mg/dl | 401±15 mg/dl | 318±58* mg/dl | 265±39** mg/dl |

| | | | | |
|---|---|---|---|---|
| †: P<0.1, *: P<0.05, **: P<0.01 | | | | |

### <Results>

As is clear from Table 1, through comparison between the initial value and the value after the five-day breeding in the PT-free group (control), the postprandial blood sugar level was found to significantly increase for the five days. In contrast, increase in the postprandial blood sugar level was found to be significantly suppressed in the 0.0001% by mass PT-containing group at a significance level of less than 10%, as compared with the PT-free group (control). Also, increase in the postprandial blood sugar level was found to be significantly suppressed in the 0.001% by mass PT-containing group at a significance level of less than 5%, as compared with the PT-free group (control). Furthermore, increase in the postprandial blood sugar level was found to be significantly suppressed in the 0.01% by mass PT-containing group at a significance level of less than 1%, as compared with the PT-free group (control).

These results indicate that use of PT in an amount of 0.0001% by mass or more gives an effect of suppressing an increase in the postprandial blood sugar level of the KKAy mice.

### (Example 2: Effect of decreasing the blood sugar level of a model mouse of hyperglycemia using PD-containing feed)

### <Method>

Panaxadiol (PD) (product of LKT Laboratories, Inc.) was mixed with a commercially available high fat diet (trade name: Quick Fat, product of CLEA Japan, Inc.) in amounts shown in the following Table 2. Each of the thus-prepared feeds was given ad libitum to model mice of hyperglycemia (KKAy mice (product of CLEA Japan, Inc.), eight weeks old, 5 mice/group) to breed them for five days (hereinafter these groups may be referred to as "PD-containing group"). Mice of a control group were given a panaxadiol (PD)-free feed (hereinafter this group may be referred to as "PD-free group").

The blood sugar level was measured at 10 a.m. before breeding of the panaxadiol (PD)-containing high fat diet (initial value) and after breeding thereof (post-breeding). CYCLIC GB SENSOR (product of Sanko Junyaku Co., Ltd.) was used for the measurement of the blood sugar level. The values shown in the following Table 2 are calculated on the basis of the average value of two values obtained by measuring each individual twice for the postprandial blood sugar level. Notably, statistical analysis was performed using the Dunnett's multiple test.

**Table 2**

| | PD-freegroup (control) | PD-containing groups | | |
|---|---|---|---|---|
| Amount of PD added | - | 0.0001% by mass | 0.001% by mass | 0.01% by mass |
| Blood sugar level (initial value) | 452±29 mg/dl | 454±38 mg/dl | 451±34 mg/dl | 454±32 mg/dl |
| Blood sugar level (post-breeding) | 530±43 mg/dl | 404±17† mg/dl | 323±56* mg/dl | 274±34** mg/dl |

| | | | | |
|---|---|---|---|---|
| †: P<0.1, *: P<0.05, **: P<0.01 | | | | |

### <Results>

As is clear from Table 2, through comparison between the initial value and the value after the five-day breeding in the PD-free group (control), the postprandial blood sugar level was found to significantly increase for the five days. In contrast, increase in the postprandial blood sugar level was found to be significantly suppressed in the 0.0001% by mass PD-containing group at a significance level of less than 10%, as compared with the PD-free group (control). Also, increase in the postprandial blood sugar level was found to be significantly suppressed in the 0.001% by mass PD-containing group at a significance level of less than 5%, as compared with the PD-free group (control). Furthermore, increase in the postprandial blood sugar level was found to be significantly suppressed in the 0.01% by mass PD-containing group at a significance level of less than 1%, as compared with the PD-free group (control).

These results indicate that use of PD in an amount of 0.0001% by mass or more gives an effect of suppressing an increase in the postprandial blood sugar level of the KKAy mice.

### (Example 3: Promotion of intake of sugar into cells derived from rat skeletal muscle by addition of PT)

### <Method>

Cells derived from rat skeletal muscle (L6 cells, product of Dainippon Pharmaceutical Co., Ltd.) were suspended in 15 mL of DMEM (Dulbecco's modified Eagle medium, product of SIGMA Co.) containing 10% by mass FBS (fetal bovine serum, product of GIBCO Co.) and 1% by mass AB (anti-biotic solution, product of SIGMA Co.). The resultant suspension was dispensed into 75-cm² culture flasks and then statically cultured at 37°C under 5%CO₂. After having reached subconfluency (80%), each culture was placed in a 24-well plate (product of SUMITOMO BAKELITE CO., LTD.) at 50,000 cells/well. After having reached confluency, the cells were cultured for three days. Then, the medium was exchanged with MEM (minimal essential medium, product of NACALAI TESQUE, INC.) containing 2% by mass FBS and 1% by mass AB to thereby induce differentiation of the L6 cells. Eight days after the induction of differentiation, a solution of panaxatriol (PT) (product of LKT Laboratories, Inc.) in ethanol was added to the resultant medium so that the concentration of panaxatriol (PT) was 1 ppm or 10 ppm. The PT-free well was used as a control for each of these wells. Then, 30 hours after, the medium of each well was exchanged with MEM containing 2% by mass BSA (bovine serum albumin, product of SIGMA Co.) and panaxatriol (PT) at a concentration of 1 ppm or 10 ppm, to thereby terminate sensitization. Furthermore, 18 hours after, the MEM containing 2% by mass BSA was removed, and a KRH buffer (50 mM HEPES (product of SIGMA Co.), pH 7.4, 137 mM sodium chloride (product of Wako Pure Chemical Industries, Ltd.), 4.8 mM potassium chloride (product of Wako Pure Chemical Industries, Ltd.), 1.85 mM calcium chloride (product of Wako Pure Chemical Industries, Ltd.) and 1.3 mM magnesium sulfate (product of Wako Pure Chemical Industries, Ltd.)) was added to the wells at 30 µL/well. Thereafter, 6 µL of RI-labeled 2-deoxyglucose (2-DG, product of American Radiolabeled Chemicals Co.) (final concentration: 6.5 mM (0.5-Cᵢ)) was added to the wells to perform reaction. Five minutes after the initiation of reaction, the cells were washed four times with an ice-cooled KRH buffer to remove 2-DG that had not been taken into the L6 cells. After removal of the KRH buffer, the cells were lysed with 250 µL of 0.05N sodium hydroxide (product of Wako Pure Chemical Industries, Ltd.) and recovered in a vial. In addition, the wells were washed twice with 200 µL of the KRH buffer and recovered in the vial similarly. Then, 3 mL of liquid scintillation cocktail PICOFLOUR (product of PerkinElmer Co., Ltd.) was added to the vial containing the cells recovered, followed by measuring for radioactivity with a liquid scintillation counter (LSC-5100, product ofAloka Co.).

Evaluation was performed with n = 4 to obtain an average value of the measurements thereof. Statistical analysis was performed using the Student's t-test in either case. Here, when P < 0.05, the results were regarded as having a significant difference therebetween. The following Table 3 shows the relative activities to the radioactivity of the PT-free well which is regarded as 100.

**Table 3**

| | PT-free (control) | PT-added | |
|---|---|---|---|
| Conc. of PT added | - | 1 ppm | 10 ppm |
| Relative activity | 100 | 132* | 145** |

| | | | |
|---|---|---|---|
| *: P<0.05, **: P<0.01 | | | |

### <Results>

As is clear from Table 3, the amount of glucose taken into the cells was found to increase by addition of PT to the L6 cells.

These results suggest that the sugar metabolism-improving agent of the present invention containing panaxatriol (PT) has an effect of promoting sugar intake in muscle, which is a central organ for intake of sugar into the body.

### (Example 4: Promotion of intake of sugar into cells derived from rat skeletal muscle by addition of PD)

### <Method>

As described in Example 3, cells derived from rat skeletal muscle (L6 cells, product of Dainippon Pharmaceutical Co., Ltd.) were suspended in 15 mL of DMEM (Dulbecco's modified Eagle medium, product of SIGMA Co.) containing 10% by mass FBS (fetal bovine serum, product of GIBCO Co.) and 1% by mass AB (anti-biotic solution, product of SIGMA Co.). The resultant suspension was dispensed into 75-cm² culture flasks and then statically cultured at 37°C under 5%CO₂. After having reached subconfluency (80%), the culture was placed in a 24-well plate (product of SUMITOMO BAKELITE CO., LTD.) at 50,000 cells/well. After having reached confluency, the cells were cultured for three days. Then, the medium was exchanged with MEM (minimal essential medium, product of NACALAI TESQUE, INC.) containing 2% by mass FBS and 1% by mass AB to thereby induce differentiation of the L6 cells. Eight days after induction of the differentiation, a solution of panaxadiol (PD) (product of LKT Laboratories, Inc.) in ethanol was added to the resultant medium so that the concentration of panaxadiol (PD) was 1 ppm or 10 ppm. The PD-free well was used as a control for each of these wells. Then, 30 hours after, the medium of each well was exchanged with MEM containing 2% by mass BSA (bovine serum albumin, product of SIGMA Co.) and panaxadiol (PD) at a concentration of 1 ppm or 10 ppm, to thereby terminate sensitization. Furthermore, 18 hours after, the MEM containing 2% by mass BSA was removed, and a KRH buffer (50 mM HEPES (product of SIGMA Co.), pH 7.4, 137 mM sodium chloride (product of Wako Pure Chemical Industries, Ltd.), 4.8 mM potassium chloride (product of Wako Pure Chemical Industries, Ltd.), 1.85 mM calcium chloride (product of Wako Pure Chemical Industries, Ltd.) and 1.3 mM magnesium sulfate (product of Wako Pure Chemical Industries, Ltd.)) was added to the wells at 30 µL/well. Thereafter, 6 µL of RI-labeled 2-deoxyglucose (2-DG, product of American Radiolabeled Chemicals Co.) (final concentration: 6.5 mM (0.5-Ci)) was added to the wells to perform reaction. Five minutes after the initiation of reaction, the cells were washed four times with an ice-cooled KRH buffer to remove 2-DG that had not been taken into the L6 cells. After removal of the KRH buffer, the cells were lysed with 250 µL of 0.05N sodium hydroxide (product of Wako Pure Chemical Industries, Ltd.) and recovered in a vial. In addition, the wells were washed twice with 200 µL of the KRH buffer and recovered in the vial similarly. Then, 3 mL of liquid scintillation cocktail PICOFLOUR (product of PerkinElmer Co., Ltd.) was added to the vial containing the cells recovered, followed by measuring for radioactivity with a liquid scintillation counter (LSC-5100, product ofAloka Co.).

Evaluation was performed with n = 4 to obtain an average value of the measurements thereof. Statistical analysis was performed using the Student's t-test in either case. Here, when P < 0.05, the results were regarded as having a significant difference therebetween. The following Table 4 shows the relative activities to the radioactivity of the PD-free well which is regarded as 100.

**Table 4**

| | PD-free (control) | PD-added | |
|---|---|---|---|
| Conc. of PD added | - | 1 ppm | 10 ppm |
| Relative activity | 100 | 130* | 140** |

| | | | |
|---|---|---|---|
| *: P<0.05, *: P<0.01 | | | |

### <Results>

As is clear from Table 4, the amount of glucose taken into the cells was found to increase by addition of PD to the L6 cells.

These results suggest that the sugar metabolism-improving agent of the present invention containing panaxadiol (PD) has an effect of promoting sugar intake in muscle, which is a central organ for intake of sugar into the body.

### (Example 5: Test of PT intake for human)

### <Measurement of sugar metabolism-related index>

### -Method-

*Panax notoginseng* powder (10 g) was suspended in 100 mL of 30% by mass aqueous solution of ethanol, and then saponins were extracted under heating for 2 hours. The obtained extract was hydrolyzed in the presence of hydrochloric acid to prepare an aglycon-containing product. The aglycon-containing product was treated with a silica gel column (SILICA GEL 60N, product of KANTO CHEMICAL CO., LTD.) to isolate and prepare panaxatriol (PT) having a purity of 99% by mass or higher.

Next, capsules each containing 8 mg of the thus-prepared panaxatriol (PT) were prepared. These capsules were given continuously for eight weeks to human subjects of a group (hereinafter may be referred to as "PT-given group") to measure the fasting blood sugar level and a change in postprandial blood sugar level which was measured after giving to the human subjects predetermined meals such as a meal containing an excessive amount of starch food (e.g., a rice ball formed of 200 g of cooked rice (350 kcal)). Also, the blood indices measured were the blood HbAlc level, the blood glycoalbumin level, the blood 1.5AG level and the fasting blood insulin level.

The blood sugar levels were measured with a simplified blood sugar meter FREESTYLE (product of NIPRO Co.). The fasting blood sugar level and the fasting insulin level were measured at 9 a.m. The postprandial blood sugar level was measured every 30 min for 120 min from 9 a.m. to 11 : 30 a.m. after the predetermined starch food had been given to the subjects. The HbA1c, glycoalbumin and 1.5AG levels were measured by a routine method of a medical facility using the blood sampled at 9 a.m.

Notably, the subjects (number: 24) were those having a fasting blood sugar level of 120 mg/dL to 140 mg/dL. The 24 subjects were randomly divided to two groups and assigned to a placebo-given group, serving as a control, and a PT-given group. Also, the results were statistically analyzed using the Student's t-test comparing the placebo-given group and the PT-given group. The time at which the capsules were given was set to 10 a.m. every day so that the capsules were not given at the same time as a meal.

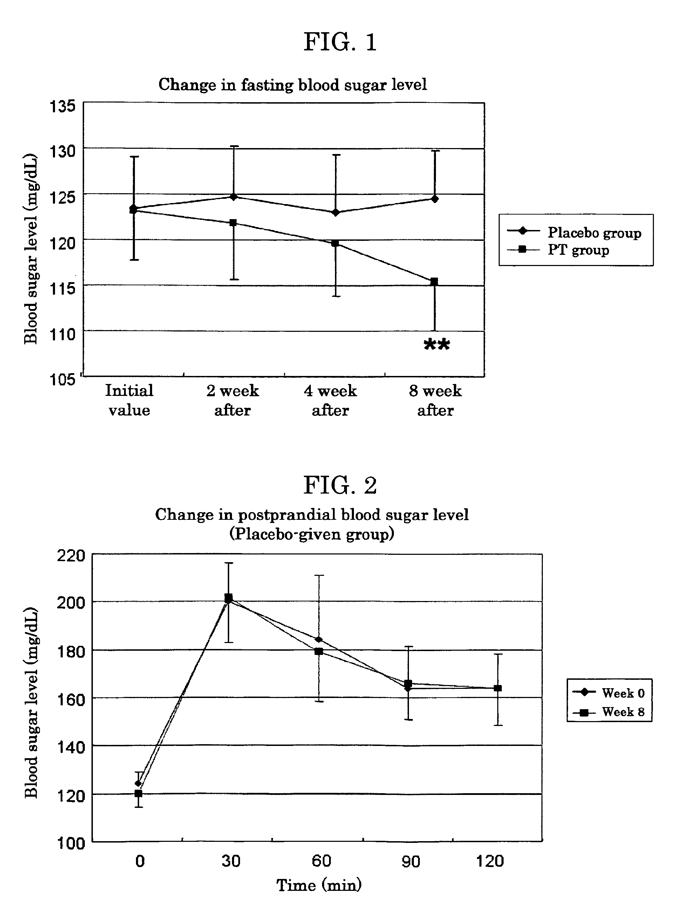

Fig. 1 shows measurement results of the fasting blood sugar level.

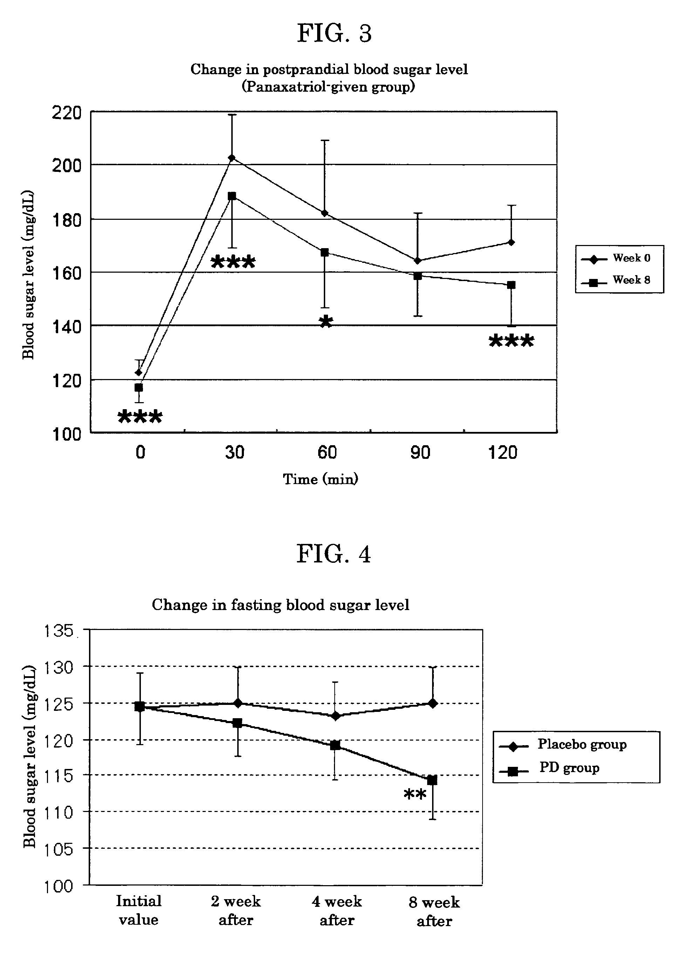

Fig. 2 shows changes in postprandial blood sugar level of the placebo-given group. Fig. 3 shows changes in postprandial blood sugar level of the PT-given group.

The following Table 5 shows measurement results of the blood indices of the placebo-given group. The following Table 6 shows measurement results of the blood indices of the PT-given group.

**Table 5**

| | Placebo-given group | | | |
|---|---|---|---|---|
| Blood indices | Week 0 | Week 2 | Week 4 | Week 8 |
| HbAlc (% by mass) | 5.1±0.1 | 5.2±0.2 | 5.2±0.1 | 5.2±0.1 |
| Glycoalbumin (% by mass) | 14.6±1.4 | 14.8±1.4 | 14.8±1.5 | 14.8±1.3 |
| 1.5AG (µg/mL) | 24.3±7.5 | 24.3±7.4 | 24.2±7.7 | 24.1±7.4 |
| Insulin (µU/mL) | 5.4±1.6 | 6.2±1.8 | 5.9±3.2 | 6.3±2.5 |

**Table 6**

| | PT-given group | | | |
|---|---|---|---|---|
| Blood indices | Week 0 | Week 2 | Week 4 | Week 8 |
| HbAlc (% by mass) | 5.1±0.1 | 5.1±0.2 | 5.1±0.1 | 4.8±0.2* |
| Glycoalbumin (% by mass) | 14.7±1.3 | 14.5±1.3** | 14.3±1.3*** | 14.2±1.3*** |
| 15AG (µg/mL) | 24.3±7.7 | 24.2±7.1 | 25.0±7.5* | 25.6±6.3** |
| Insulin (µU/mL) | 5.5±1.5 | 6.1±2.1 | 5.8±4.2 | 6.2±2.4 |

| | | | | |
|---|---|---|---|---|
| *: P<0.05, **: P<0.01, ***: P<0.001 | | | | |

### -Results-

As a result of the test of PT intake for human (Example 5), it was found that the fasting blood sugar level of the PT-given group decreased significantly as compared with the placebo-given group (Fig. 1). The postprandial blood sugar level of the PT-given group was found to decrease significantly as compared with the placebo-given group 30 min, 60 min and 120 min after the PT had been given, indicating that the sugar metabolism improving effects were clearly observed (Figs. 2 and 3).

These results clearly indicate that the diabetes-improving drug of the present invention containing panaxatriol (PT) reduces both of the fasting blood sugar level and the postprandial blood sugar level.

Also, as shown in Tables 5 and 6, through measurement of the blood indices relating to sugar metabolism, the HbAlc level of the PT-given group was found to decrease significantly as compared with the placebo-given group eight weeks after the PT had been given, and the glycoalbumin level of the PT-given group was found to decrease significantly as compared with the placebo-given group. In addition, a significant decrease in the glycoalbumin level was observed between Week 0 and Week 8 in the PT-given group. The 1.5AG level of the PT-given group was found to increase after Week 4. There was no statistically significant change in the fasting insulin level of the PT-given group. Thus, presumably, the reason why panaxatriol (PT) exhibited the effect of suppressing an increase in the blood sugar level is not that it enhanced insulin secreting capability but that it increased insulin sensitivity.

These results indicate that it is not necessary to take in the diabetes-improving drug of the present invention containing panaxatriol (PT) at the same time as a meal. Also, advantageous effects were obtained even when it was given once a day. These advantages are thought to be meaningful in facilitating continuous intake.

### (Example 6: Test of PD intake for human)

### <Measurement of sugar metabolism-related index>

### -Method-

*Panax notoginseng* powder (10 g) was suspended in 100 mL of 30% by mass aqueous solution of ethanol, and then saponins were extracted under heating for 2 hours. The obtained extract was hydrolyzed in the presence of hydrochloric acid to prepare an aglycon-containing product. The aglycon-containing product was treated with a silica gel column (SILICA GEL 60N, product of KANTO CHEMICAL CO., LTD.) to isolate and prepare panaxadiol (PD) having a purity of 99% by mass or higher.

Next, capsules each containing 8 mg of the thus-prepared panaxadiol (PD) were prepared. These capsules were given continuously for eight weeks to human subjects of a group (hereinafter may be referred to as "PD-given group") to measure the fasting blood sugar level and a change in postprandial blood sugar level which was measured after giving to the human subjects predetermined meals such as a meal containing an excessive amount of starch food (e.g., a rice ball formed of 200 g of cooked rice (350 kcal)). Also, the blood indices measured were the blood HbAlc level, the blood glycoalbumin level, the blood 1.5AG level and the fasting blood insulin level.

The blood sugar levels were measured with a simplified blood sugar meter FREESTYLE (product of NIPRO Co.). The fasting blood sugar level and the fasting insulin level were measured at 9 a.m. The postprandial blood sugar level was measured every 30 min for 120 min from 9 a.m. to 11 : 30 a.m. after the predetermined starch food had been given to the subjects. The HbAlc, glycoalbumin and 1.5AG levels were measured by a routine method of a medical facility using the blood sampled at 9 a.m.

Notably, the subjects (number: 24) were those having a fasting blood sugar level of 120 mg/dL to 140 mg/dL. The 24 subjects were randomly divided to two groups and assigned to a placebo-given group, serving as a control, and a PD-given group. Also, the results were statistically analyzed using the Student's t-test comparing the placebo-given group and the PD-given group. The time at which the capsules were given was set to 10 a.m. every day so that the capsules were not given at the same time as the meal.

Fig. 4 shows measurement results of the fasting blood sugar level.

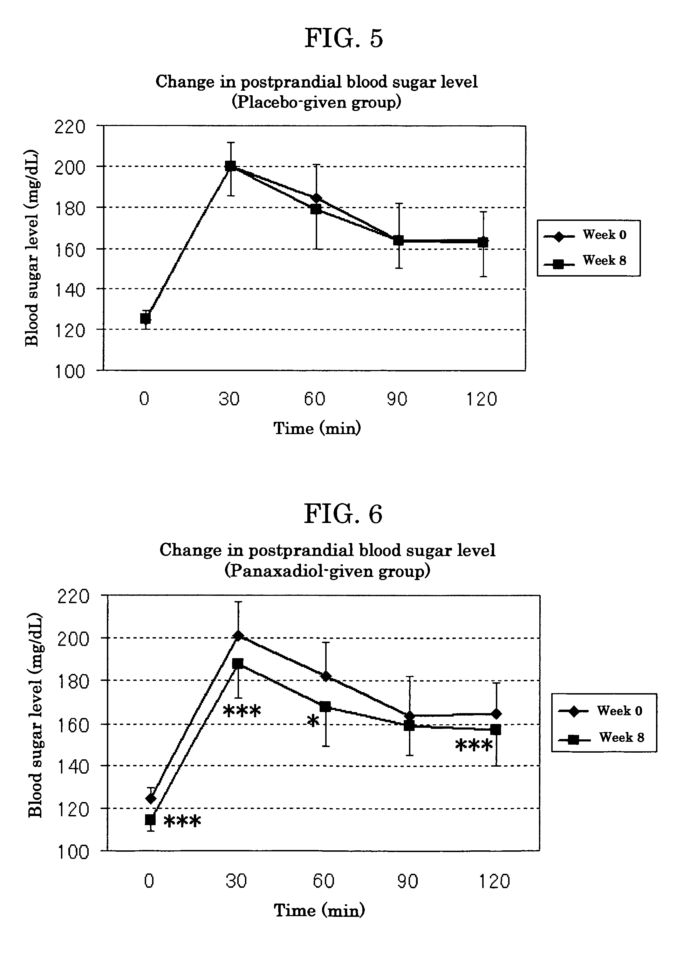

Fig. 5 shows changes in postprandial blood sugar level of the placebo-given group. Fig. 6 shows changes in postprandial blood sugar level of the PD-given group.

The following Table 7 shows measurement results of the blood indices of the placebo-given group. The following Table 8 shows measurement results of the blood indices of the PD-given group.

**Table 7**

| | Placebo-given group | | | |
|---|---|---|---|---|
| Blood indices | Week 0 | Week 2 | Week 4 | Week 8 |
| HbAlc (% by mass) | 5.1±0.1 | 5.2±0.2 | 5.2±0.1 | 5.2±0.1 |
| Glycoalbumin (% by mass) | 14.6±1.4 | 14.8±1.4 | 14.8±1.5 | 14.8±1.3 |
| 1.5AG (µg/mL) | 24.3±7.5 | 24.3±7.4 | 24.2±7.7 | 24.1±7.4 |
| Insulin (µU/mL) | 5.4±1.6 | 6.2±1.8 | 5.9±3.2 | 6.3±2.5 |

**Table 8**

| | PD-given group | | | |
|---|---|---|---|---|
| Blood indices | Week 0 | Week 2 | Week 4 | Week 8 |
| HbA1c (% by mass) | 5.1±0.1 | 5.1±0.2 | 5.1±0.1 | 4.8±0.1* |
| Glycoalbumin (% by mass) | 14.8±1.4 | 14.5±1.2** | 14.3±1.3*** | 14.2±1.1*** |
| 1.5AG (µg/mL) | 24.4±7.8 | 24.1±7.2 | 25.0±7.3* | 25.7±6.4** |
| Insulin (µU/mL) | 5.6±1.4 | 5.9±2.2 | 5.9±3.8 | 6.1±2.6 |

| | | | | |
|---|---|---|---|---|
| *: P<0.05, **: P<0.01, ***: P<0.001 | | | | |

### -Results-

As a result of the test of PD intake for human (Example 6), it was found that the fasting blood sugar level of the PD-given group decreased significantly as compared with the placebo-given group (Fig. 4). The postprandial blood sugar level of the PD-given group was found to decrease significantly as compared with the placebo-given group 30 min, 60 min and 120 min after the PD had been given, indicating that the sugar metabolism improving effects were clearly observed (Figs. 5 and 6).

These results clearly indicate that the diabetes-improving drug of the present invention containing panaxadiol (PD) reduces both of the fasting blood sugar level and the postprandial blood sugar level.

Also, as shown in Tables 7 and 8, through measurement of the blood indices relating to sugar metabolism, the HbAlc level of the PD-given group was found to decrease significantly as compared with the placebo-given group eight weeks after the PD had been given, and the glycoalbumin level of the PD-given group was found to decrease significantly as compared with the placebo-given group. In addition, a significant decrease in the glycoalbumin level was observed between Week 0 and Week 8 in the PD-given group. The 1.5AG level of the PD-given group was found to increase after Week 4. There was no statistically significant change in the fasting insulin level of the PD-given group. Thus, presumably, the reason why panaxadiol (PD) exhibited the effect of suppressing an increase in the blood sugar level is not that it enhanced insulin secreting capability but that it increased insulin sensitivity

These results indicate that it is not necessary to intake the diabetes-improving drug of the present invention containing panaxadiol (PD) at the same time as a meal. Also, advantageous effects were obtained even when it was given once a day. These advantages are thought to be meaningful in facilitating continuous intake.

### (Example 7: Effect of promoting metabolism of sugar derived from meals)

Twenty-four model mice of hyperglycemia (KKAy mouse (product of CLEA Japan, Inc.), male, four weeks old) were preliminarily bred for one week in individual cages under the following conditions: temperature: room temperature (22°C ± 1°C), humidity: 50% ± 5%, and light-dark cycle: 12 hours. Eight grams per day of CE-2 (product of CLEA Japan, Inc.) was given to each mouse as feed during the preliminary breeding, while water was given ad libitum.

After completion of the preliminary breeding, the mice were measured for body weight and blood sugar level. Then, they were divided into three groups, each containing eight mice, so that the body weights and blood sugar levels were averaged in the three groups, followed by main breeding in a manner described below. Notably, the blood sugar level of each mouse was measured with CYCLIC GB SENSOR (product of Sanko Junyaku Co., Ltd.) using the whole blood sampled from the tale vein of the mouse.

### -Control group-

The mice of one group were subjected to main breeding for one week under the conditions that eight grams per day of a commercially available high fat diet (trade name: Quick Fat, product of CLEA Japan, Inc.) was given to each mouse while water was given ad libitum. Hereinafter, this group may be referred to as "control group."

### -PT-administered group-

The mice of another group was subjected to main breeding for one week under the conditions that eight grams per day of a commercially available high fat diet (trade name: Quick Fat, product of CLEA Japan, Inc.) containing 0.1% by mass panaxatriol (PT) (product of LKT Laboratories, Inc.) was given to each mouse while water was given ad libitum. Hereinafter, this group may be referred to as "PT-administered group."

### -PD-administered group-

The mice of the other group were subjected to main breeding for one week under the conditions that eight grams per day of a commercially available high fat diet (trade name: Quick Fat, product of CLEA Japan, Inc.) containing 0.1% by mass panaxadiol (PD) (product of LKT Laboratories, Inc.) was given to each mouse while water was given ad libitum. Hereinafter, this group may be referred to as "PT-administered group."

After the one-week main breeding, the mice were measured for blood sugar level and body weight, and then were subjected to measurement of the amount of ¹³CO₂ excreted. The blood sugar level was found to be 335.4 mg/dL in the control group, 199.3 mg/dL in the PT-administered group, and 231.6 mg/dL in the PD-administered group.

### <Measurement of amount of ¹³CO₂ excreted>

The mice were transferred to an exhaled gas analysis chamber (ARCO-2000-ISO System, product of ARCO SYSTEM Co.) where they were conditioned for 2 hours. Thereafter, an aqueous solution of glucose and glucose-U-¹³C₆ was orally administered to each mouse at a concentration calculated from the following: [2.0 g D-glucose (product of NACALAI TESQUE, INC.) + 72 mg D-glucose-U-¹³C₆ (99%) (product of Cambridge Isotope Lab.)/kg-body weight]. Then, the cumulative amount of ¹³CO₂ excreted was measured for four hours. The cumulative amount of ¹³CO₂ excreted is expressed as AUC (increment in the amount of ¹³CO₂ excreted). Here, the AUC (increment in the amount of ¹³CO₂ excreted) refers to an area corresponding to the increment between the amount of ¹³CO₂ excreted prior to administration of glucose-U-¹³C₆ (baseline) to the amount of ¹³CO₂ excreted measured four hours after administration of glucose-U-¹³C₆ in a graph where a change in the amount of ¹³CO₂ excreted is plotted against time after administration of glucose-U-¹³C₆.

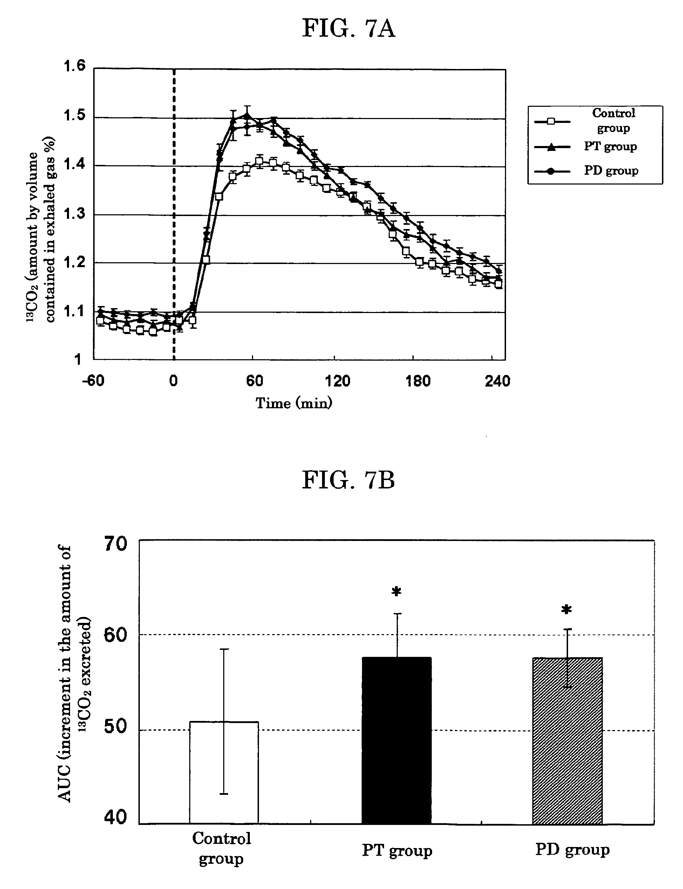

Fig. 7A shows changes over time in the amount of ¹³CO₂ excreted, and Fig. 7B shows the cumulative amounts of ¹³CO₂ excreted. These results indicate that the amount of ¹³CO₂ excreted significantly increased in the PT-administered group and PD-administered group (statistical analysis was performed using the Student's t-test, and when P < 0.05, the results were regarded as having a significant difference therebetween). That is, it was found that PT or PD promoted metabolism of sugar supplied from meals.

### Industrial Applicability

The sugar metabolism-improving agent of the present invention containing at least one of panaxatriol (PT) and panaxadiol (PD) and the sugar metabolism-improving composition containing the sugar metabolism-improving agent have an excellent effect of promoting intake of sugar into muscle cells, an excellent effect of suppressing an increase in the postprandial blood sugar level, an excellent effect of decreasing the fasting blood sugar level, an excellent effect of controlling the sugar metabolism-related indices, and an excellent effect of promoting metabolism of sugar derived from meals. Thus, they can suitably be used as an agent for suppressing an increase in the postprandial blood sugar level, an agent for decreasing the fasting blood sugar level, and an agent for promoting sugar intake, and are effective to the prevention or treatment of diabetes

Furthermore, the sugar metabolism-improving agent has high safety and can suitably be used in food and drink.

## Claims

1. A sugar metabolism-improving agent comprising:
at least one of a compound having a structure expressed by the following Structural Formula (1) and a compound having a structure expressed by the following Structural Formula (2).

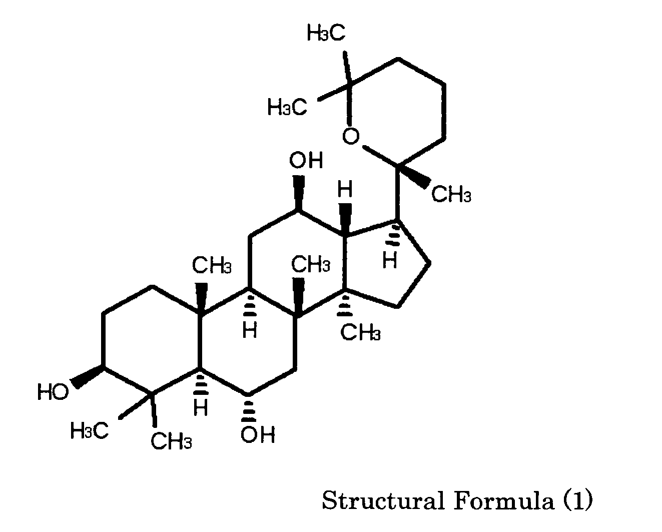

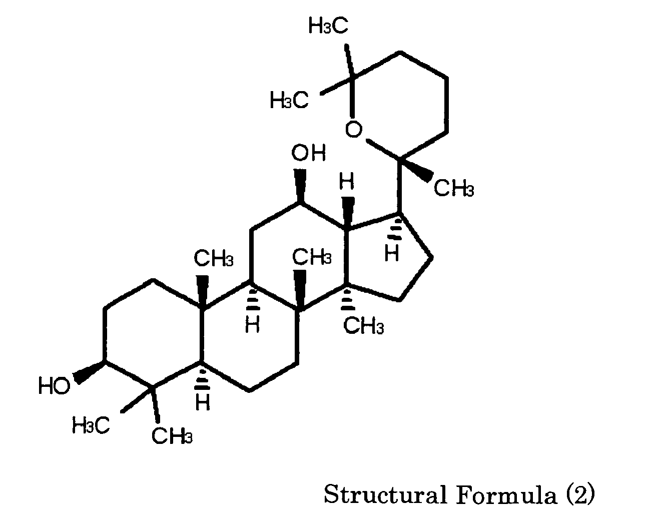

2. The sugar metabolism-improving agent according to claim 1, wherein the sugar metabolism-improving agent has an effect of suppressing an increase in a postprandial blood sugar level.

3. The sugar metabolism-improving agent according to claim 1 or 2, wherein the sugar metabolism-improving agent has at least one of an effect of decreasing a fasting blood sugar level and an effect of controlling a sugar metabolism-related index in serum.

4. The sugar metabolism-improving agent according to any one of claims 1 to 3, wherein the sugar metabolism-improving agent has an effect of promoting metabolism of sugar derived from a meal.

5. The sugar metabolism-improving agent according to any one of claims 1 to 4, wherein the sugar metabolism-improving agent exhibits a sugar metabolism-improving effect regardless of whether or not the sugar metabolism-improving agent is given at the same time as a meal.

6. The sugar metabolism-improving agent according to any one of claims 1 to 5, wherein an amount of the sugar metabolism-improving agent given per day is at least 1 mg.

7. A sugar metabolism-improving composition comprising:
the sugar metabolism-improving agent according to any one of claims 1 to 6.
